# EUROPEAN PATENT SPECIFICATION

(11) **EP 4 257 100 B1**
(45) Date of publication and mention of the grant of the patent: **16.10.2024**
(21) Application number: 22166961.7
(22) Date of filing: 06.04.2022
(51) Int. Cl.: A61F 13/02, A61B 17/02

(54) **A MEDICAL DRESSING FOR USE IN CONJUNCTION WITH A TROCAR**
MEDIZINISCHER VERBAND ZUR VERWENDUNG IN VERBINDUNG MIT EINEM TROKAR
PANSEMENT MÉDICAL DESTINÉ À ÊTRE UTILISÉ CONJOINTEMENT AVEC UN TROCART

(43) Date of publication of application: 11.10.2023
(73) Proprietor: Mölnlycke Health Care AB, 402 52 Göteborg (SE)
(72) Inventor: ROSENGREN, Oscar, Hovås (SE); VALHAM, David, Västra Frölunda (SE)
(74) Representative: Kransell & Wennborg KB

(56) References cited:
- WO-A1-94/28951
- US-A- 6 048 309
- US-A1- 2005 159 711
- US-A1- 2017 049 474
- US-A1- 2021 307 740

## Description

### TECHNICAL FIELD

The present disclosure generally relates to a medical dressing for use in conjunction with a trocar. The present disclosure also relates to a trocar system comprising the medical dressing.

### BACKGROUND

Minimal invasive surgery (MIS) is a technique for performing surgery that is associated with less pain, shorter recovery and fewer complications compared to traditional open surgery.

In an MIS procedure, a small incision is typically made through the skin of a patient, and a trocar is thereafter inserted into the incision. The trocar comprises a cannula; i.e. a hollow and tube-shaped shaft that is placed inside the patient to provide access to the body cavity during the MIS procedure. The cannula serves as a working channel that allows for the entry of various surgical instruments and cameras into a body cavity, e.g. an abdominal cavity.

During an MIS procedure, a plurality of surgical instruments and tools are typically utilized. One challenge during MIS procedures is that the trocar may be dislocated during surgery; i.e. that the trocar may slip out from the body cavity or that the trocar projects too deep into the body cavity. This may result in surgical failure and is also associated with risks for the patient.

To date, commonly used trocars in MIS procedures include balloon trocars, and Hasson trocars, respectively. These trocar types prevent the problem of trocar dislocation in different manners. A Hasson trocar is a non-bladed trocar that stays in place by means of sutures anchored to the abdominal wall fascia. A balloon trocar comprises a balloon at the end of the trocar that is inflated with air. Instead of utilizing sutures for fixing and keeping the trocar in place, the inflated balloon anchors the trocar in the abdomen.

Both the balloon and Hasson trocar system suffer from disadvantages. For example, the inflated balloon of a balloon trocar may rupture, which is associated with risks for the patient. Furthermore, a balloon trocar cannot simply be removed from the body cavity since the balloon must first be deflated. The Hasson trocar is cumbersome in the sense that fixation and stabilization require the aid of sutures to keep the trocar in place. Upon removal of the trocar, the sutures must first be removed. The prior art documents US2017049474 and US2005159711 disclose medical dressing to be used with a trocar.

Another challenge during MIS procedures is sterility and keeping the surgical sites and the areas circumventing the trocars clean and free from blood and contaminants.

In view of this, there is a need to provide an improved, safe and simplified means to facilitate the MIS procedure for the surgical staff such that trocar dislocation and contamination are prevented during surgery.

### SUMMARY

In view of the above-mentioned problems, it is an object of the present disclosure to provide improvements with respect to relieving the burden for the surgical staff and providing a facilitated and improved means to handle and operate a trocar during minimal invasive surgery.

According to a first aspect, there is provided a medical dressing for use in conjunction with a trocar, wherein the dressing has a distal side provided with a backing layer and an opposing proximal side provided with an adhesive skin contact layer; the dressing comprising a centrally disposed aperture for receiving a trocar during use, wherein the aperture extends through the backing layer and the adhesive skin contact layer; the dressing further comprising at least a first flange and a second flange configured to project upwardly from the distal side of the dressing; wherein each one of the at least first and second flanges comprises a respective proximal end portion being attached to the dressing and a respective distal end portion configured to be adhesively attached to a trocar during use.

The present disclosure is based on the realization that a medical dressing as defined hereinabove greatly facilitates the minimally invasive surgical (MIS) procedure for the surgeon and for the surgical staff. A simplified means for stabilizing the trocar is provided, and contamination as well as undesired trocar dislocation during use are prevented.

The dressing may initially be attached to the skin of a patient in an area where the MIS procedure is to be accomplished. When the dressing has been attached, the surgeon may puncture the skin, through the aperture of the dressing, and subsequently insert a trocar through the punctured skin site.

The distal end portions of the at least first and second flanges may be adhesively attached to the trocar, more particularly to the cannula of the trocar. In use, and in a rested or relaxed configuration, the flanges prevent the trocar from slipping out of the body cavity.

In many situations, the trocar needs to be tilted and inserted deeper into the body cavity to access a particular part of the body cavity. The dressing of the present disclosure facilitates the access of such parts during surgery, but prevents the trocar from being inserted too deep into the body cavity.

The medical dressing of the present disclosure has a simple construction and provides for a safe and facilitated means to operate and stabilize the trocar. The dressing, when used in conjunction with a trocar, does not require any additional trocar fixation part arranged inside the body (as is the case with balloon trocars) or fixation by means of sutures to the skin (as is the case with Hasson trocars). Furthermore, the attachment and detachment of the dressing and the trocar is significantly facilitated.

The adhesive skin contact layer of the dressing secures a tight fit to the skin, and the dressing is fixed in place in the area circumventing the surgical site. Blood and body fluids exuded from the surgical site may be evaporated through the backing layer.

The aperture of the dressing is typically dimensioned to receive a trocar. The length and/or diameter of the aperture may correspond to the diameter of a trocar cannula. Typically, the length and/or diameter of the aperture is slightly larger than the diameter of a trocar cannula.

After surgery, when the trocar is to be exerted from the body cavity, the flanges may be detached from the trocar, and the dressing may subsequently be removed after the trocar has been removed.

The flanges allow the trocar to be held in a straight configuration with respect to the incision site, but also allow the trocar to be moved angularly; i.e. the trocar may adopt a slanted configuration. The surgeon may thus access a particular organ or part of the body cavity in a more flexible manner. The attachment of the distal end portions of the flanges prevents the trocar from being slanted too much, and the risk of harmful intervention in the body cavity is thereby decreased.

Each one of the flanges may comprise a foldable material.

This allows for the flanges to adopt an extended configuration and a bent and/or folded configuration.

In the extended configuration, the flanges have a straight extension and restrict the trocar from sliding out of the body cavity. The flanges secure that the trocar remains inside the body cavity during the surgical procedure.

In the bent configuration, the flanges are bent and/or slightly folded, which allows for the trocar to project deeper into the body cavity. This may be desired in scenarios where a particular part of an organ residing in the body cavity is to be reached. The flanges secure that the trocar does not project too deep into the body cavity.

The flanges should not be too rigid or stiff as this would prevent the flanges from following the movement of the trocar. The flanges thus preferably comprise a foldable material; i.e. a flexible or bendable material.

In exemplary embodiments, the dressing further comprises at least a third, e.g. at least a fourth flange projecting upwardly from the distal side of the dressing.

The additional flanges provide for an improved stability of the trocar and an improved means to prevent the trocar from dislocation.

The flanges may also be adhered to the trocar cannula at different positions along the longitudinal extension of the cannula. For example, in some scenarios it may be desired to insert and position the trocar in an angled or tilted configurations. Accordingly, the surgical staff may flexibly adapt the medical dressing, by means of the flanges, depending on the specific surgical procedure and scenario.

In exemplary embodiments, each one of the flanges comprises a middle portion arranged between the proximal end portion and the distal end portion, wherein the middle portion and the distal end portion form a respective projecting flange portion being unattached to the dressing.

The proximal end portions of the flanges are attached to the dressing.

The projecting flange portions are configured to extend upwardly from the distal side of the dressing. The projecting flange portions may also be folded towards the backing layer such that the dressing can be stored and packaged in a flat condition.

In exemplary embodiments, the distal end portion of each one of the flanges comprises an adhesive coating

The adhesive coating allows for the flange to be adhesively attached to a trocar cannula during use. The distal end portion is typically detachably attached to the trocar cannula. Accordingly, when the surgery is completed, the distal end portions may be detached from the trocar.

Each one of the flanges may further comprise a release liner portion attached to the adhesive coating of the distal end portion.

The release liner portions serve to protect the adhesive coating from contamination and from losing adhesiveness prior to use. The release liner portions are removed prior to attachment of the flanges to the trocar.

In exemplary embodiments, the middle portion of each one of the flanges is non-adhesive.

Accordingly, only the distal end portion is adhesively attached to the trocar; i.e. trocar cannula. The middle portion is preferably not adhesively attached to the trocar. This is to enable the flanges to bend and follow the movement of the trocar, and thereby shift between an extended and a bent configuration.

Preferably, the flanges are disposed symmetrically along the perimeter of the aperture.

Accordingly, the flanges are separated from one another, and the distance between each respective flange may be the same. This increases the stability of the trocar and facilitates the operation of the trocar during surgery.

Typically, each projecting flange portion is configured to extend from a position along the perimeter of the aperture of the dressing.

The length, l, of each one of the projecting flange portion may be in the range of from 20 to 70 mm, preferably from 25 to 50 mm.

The length refers to the total length of the distal and middle portion of the flange. The length of the projecting flange portion is thus measured from the distal side of the dressing to a distal end point of the flange.

A length, l, in the above-mentioned ranges allows for the trocar to be angled and moved into and inside the body cavity of a patient. It also restricts the trocar from penetrating too deep into the body cavity and from slipping out of the body cavity.

The proximal end portion of each one of the flanges may be attached to the backing layer of the dressing. Alternatively, it may be attached to the dressing between the backing layer and the adhesive skin contact layer. In the latter case, the projecting flange portion may extend through the aperture and project upwardly from the distal side of the dressing.

In exemplary embodiments, the aperture is covered by an incision film configured to be cut prior to or during insertion of a trocar into the aperture.

An incision film is a film which is designed to be cut through during surgery. The incision film prevents the incision site from bacterial contamination and seals the area around the trocar cannula. Prior to surgery, the body cavity, e.g. the abdomen is typically insufflated with a gas, typically carbon dioxide, to produce a pneumoperitoneum. Creating a pneumoperitoneum allows for separation between the body wall and internal organs and thereby allows for an internal working space for manipulation of organs by surgical instruments.

It is important to prevent gas from leaking out of the body. The provision of an incision film may prevent undesired leakage of gas from the incision site.

In exemplary embodiments, the incision film comprises markings indicating where and/or how to cut the incision film.

When inserting a trocar into the skin of a patient, it is important that the length of the incision matches that of the trocar diameter. Otherwise, there is an enhanced risk for the trocar to slip out of the body cavity. Furthermore, there is an enhanced risk for contamination. There is also a risk for leakage of gas, e.g. carbon dioxide from the body cavity.

A common practice during MIS procedures is that the surgical staff, prior to surgery, presses the trocar onto the skin at the desired insertion point to create a temporary marking on the skin. The surgeon can thereafter use that marking to determine the boundaries for the incision. By providing markings onto the incision film, a direct guidance is given as to where and/or how to cut the incision film to secure a correct size of the incision. The markings match the diameter of commonly used trocars. Accordingly, the risk of making a too large or too small incision prior to trocar insertion is minimized.

The adhesive skin contact layer of the dressing may comprise any adhesive. Preferably a skin-compatible adhesive is utilized.

In exemplary embodiments, the adhesive skin contact layer comprises a silicone-based adhesive.

A silicone-based adhesive is skin-friendly and gentle to the skin. The silicone-based adhesive allows for the dressing to be removed in a gentle manner from the skin without causing trauma and without interfering with the incision

In exemplary embodiments, the medical dressing comprises an absorbent pad between the backing layer and the adhesive skin contact layer.

An absorbent pad is beneficial to absorb the blood arising from the surgical intervention. It is desirable to prevent blood from accumulating at the skin, and instead secure removal and proper handling of the blood and body fluids by means of the absorbent pad. Furthermore, contaminating microorganisms are prevented from accumulating at the skin, and the sterility is thereby improved.

According to another aspect, there is provided a trocar system comprising the medical dressing as described hereinbefore and a trocar.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1a illustrates a medical dressing according to an exemplary embodiment of the present disclosure.
Figure 1b illustrates a medical dressing according to an exemplary embodiment of the present disclosure, wherein the aperture is covered with an incision film.
Figure 2a illustrates a medical dressing according to an exemplary embodiment of the present disclosure prior to attachment of the dressing to a trocar.
Figure 2b illustrates a medical dressing according to an exemplary embodiment of the present disclosure in use and wherein the distal end portions of the flanges of the dressing are attached to a trocar.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

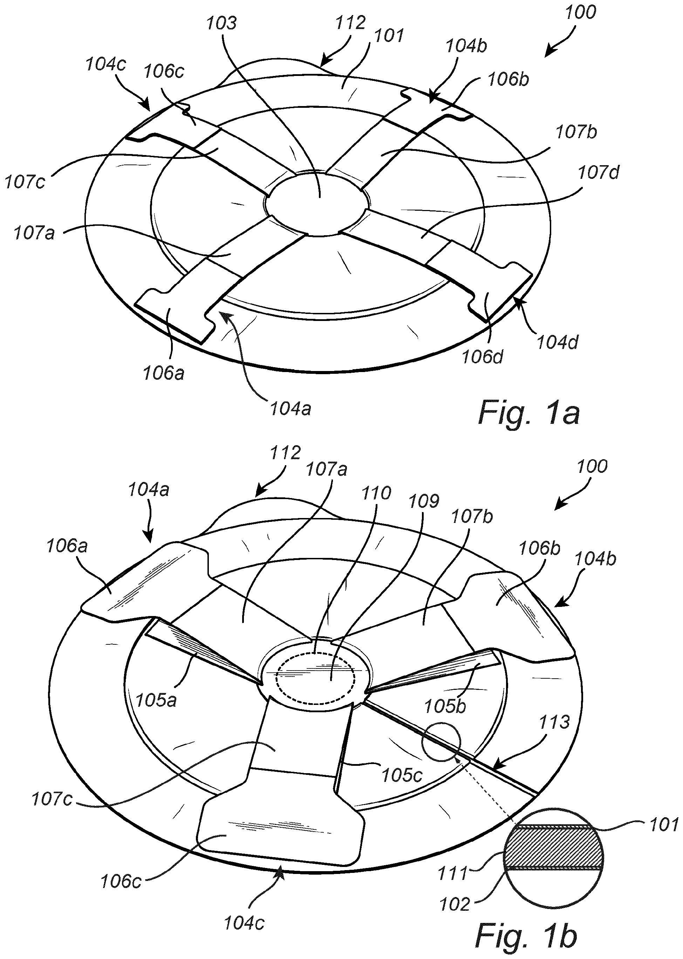

With reference to figures 1a and 1b, medical dressings 100 for use in conjunction with a trocar, according to exemplary embodiments of the present disclosure, are conceptually illustrated.

The dressing 100 has a distal side provided with a backing layer 101 and an opposing proximal side provided with an adhesive skin contact layer 102; the dressing 100 comprising a centrally disposed aperture 103 for receiving a trocar during use, wherein the aperture 103 extends through the backing layer 101 and the adhesive skin contact layer 102; the dressing 100 further comprising at least a first flange 104a and a second flange 104b configured to project upwardly from the distal side of the dressing; wherein each one of the at least first 104a and second 104b flanges comprises a respective proximal end portion (105a-c in figure 1b) being attached to the dressing 100 and a respective distal end portion 106a-d configured to be adhesively attached to a trocar during use.

The "distal side" of the dressing is the side facing away from the skin of the patient.

The "proximal side" of the dressing is the side facing and being arranged in contact with the skin of a patient.

The "backing layer" is the top layer of the dressing. The backing layer may e.g. comprise a polymeric film, e.g. a polyethylene film, a polypropylene film or a polyurethane film. Typically, the backing layer comprises a polyurethane film. The thickness of the backing layer may be in the range of from 10 to 50 µm, e.g. from 15 to 40 µm.

As used herein, the term "adhesive skin contact layer" means a layer configured to detachably adhere the dressing to a dermal surface. In other words, the adhesive skin contact layer is configured to contact the skin of patient.

The aperture 103 of the dressing is dimensioned to receive a trocar. The aperture is not limited to a specific shape. Typically, the aperture is circular. The diameter of the aperture may correspond to the diameter of a trocar cannula. Typically, the diameter of the aperture is slightly larger than the diameter of a trocar cannula. For example, the aperture may have a diameter of from 6 to 35 mm, e.g. from 10 to 30 mm, e.g. from 15 to 25 mm.

The term "flange" means a projecting piece or strip of material extending from the distal side of the dressing and being configured to attach the dressing to a trocar during use. The flanges act as "arms" connecting the dressing to the cannula of a trocar. The at least first and second flanges stabilize the trocar and prevent it from projecting out of the body cavity. The flanges also prevent the trocar from projecting too deep into the body cavity to avoid harming the organs disposed therein.

The shape of the flanges may be rectangular, tapered, conical, curved, angular or any other shape or combination of shapes. The flanges are not limited to a specific shape, but any shape may be used. The size of the flanges may vary. The surface area of a flange may correspond to 5 to 25 %, e.g. from 5 to 15% of the total surface area of the dressing.

Projecting "upwardly" means projecting in a direction away from the distal side of the dressing. The flanges project at an angle from the plane formed by the distal side of the dressing. The flanges may project in a direction substantially perpendicular to the distal side of the dressing.

To enhance the stability of the trocar during use and to secure proper anchoring to a trocar during use, it may be desired that the dressing comprises at least three, e.g. at least four flanges.

The medical dressing illustrated in figure 1a comprises four flanges 104a-d. The medical dressing illustrated in figure 1b comprises three flanges 104a-c.

The flanges 104a-d typically comprise a foldable material.

As used herein, the term "foldable material" means a material that can be bent and/or folded and follow the movement of a trocar downwards (i.e. deeper into the body cavity) and upwards (i.e. in a direction toward the incise site). The flanges should not be too rigid or stiff as this would prevent the flanges from following the movement of the trocar.

Each one of the flanges may adopt a folded and/or bent configuration and an extended configuration.

In exemplary embodiments, the foldable material may be selected from a nonwoven, a rubber, an elastomeric material, a foam, and/or a polymeric film.

As illustrated in figure 1a, the flanges 104a-d may be configured to be folded against the backing layer 101 prior to use. This enables the dressing to be stored and packaged in a substantially flat condition.

Each one of the flanges 104a-d comprises at least a proximal end portion and a distal end portion 106a-d. The proximal end portions of the flanges (denoted 105a-c in figure 1b) are attached to the dressing, and the distal end portions 106a-d are to be attached to a trocar cannula during use. Each flange 104a-d may also comprises a middle portion 107a-d between the proximal end portion 105a-c and the distal end portion 106a-d. The middle portion 107a-d and the distal end portion 106a-d form a respective projecting flange portion being unattached to the dressing.

The projecting flange portions (formed by the middle portions and distal end portions of the flanges) are configured to extend upwardly from the distal side of the dressing. The projecting flange portions may project away from the plane formed by the distal side of the dressing.

The proximal end portions 105a-c of the flanges 104a-d may be attached to the dressing by any means, e.g. by adhesion or lamination. For example, the proximal end portions 105a-c of the flanges 104a-d may be adhesively attached to the backing layer (as best illustrated in figure 1b). The proximal end portions of the flanges 104a-d may also be attached to the dressing between the backing layer 101 and the adhesive body contact layer 102. In such embodiments, the projecting flange portions (106a-d, 107a-d) may project through at least a portion of the aperture 103 and upwardly from the distal side of the dressing.

As illustrated in figure 1b, the proximal end portions 105a-c may be attached to the backing layer 101, and the flanges extend upwardly from the distal side of the dressing.

The projecting flange portions typically project from the backing layer in proximity of the aperture. For example, the projecting flange portions may extend upwardly from at least a first and a second position along the perimeter of the aperture.

The backing layer 101 may comprise an inner region surrounding the aperture, and an outer region surrounding the inner region, wherein the inner region constitutes less than 20%, preferably less than 10% of the surface area of the backing layer 101, and wherein each one of the flanges 104a-d is configured to extend upwardly from the inner region of the backing layer 101.

Preferably, the flanges 104a-d are disposed symmetrically along the perimeter of the aperture 103.

If the dressing is substantially circular, the flanges are typically radially symmetrical about a central point of the dressing.

The flanges generally have the same size and shape and are arranged regularly around the aperture of the dressing.

Typically, the distal end portion 106a-d of each one of the flanges 104a-d comprises an adhesive coating.

The adhesive coating is not limited to a specific adhesive, but any adhesive may be utilized. For example, the adhesive coating may comprise a polyacrylate or a silicone-based adhesive.

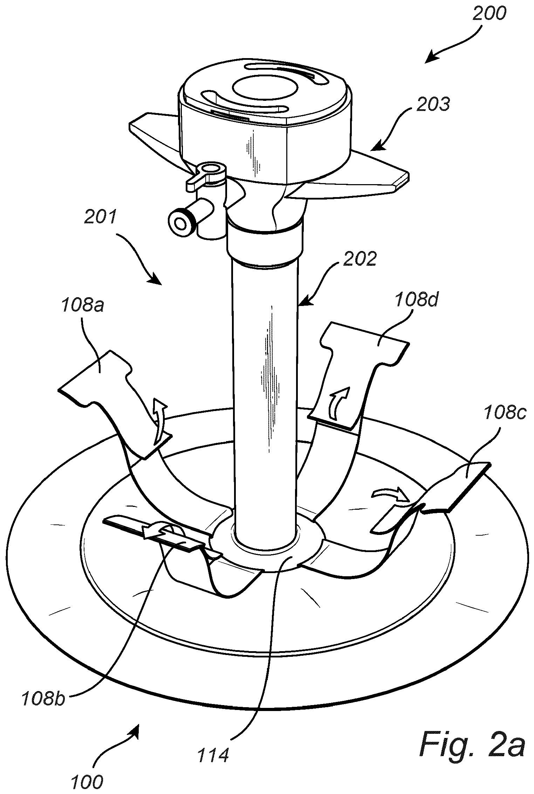

As best illustrated in figure 2a, each one of the flanges 104a-d may further comprise a release liner portion 108a-d attached to the adhesive coating of the distal end portions 106a-d.

The release liner portions may e.g. comprise a material selected from polyethylene, polyester, polypropylene and silicone coated paper. For example, the release liner may be a polyethylene film having a thickness in the range of from 30 to 300 µm, e.g. from 50 to 150 µm.

The release liner portions 108a-d are removed prior to use (as illustrated by the arrows in figure 2a) and the adhesive coating of the distal end portions allow for the flanges to be adhesively attached to the trocar cannula 202.

Typically, the middle portion 107a-d of each one of the flanges 104a-d is non-adhesive.

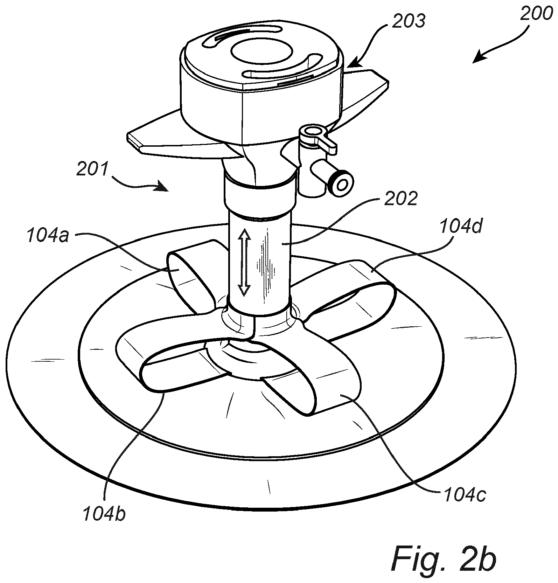

This is to facilitate the flanges to adopt a bent/folded configuration and follow the movement of the trocar in the longitudinal direction; i.e. upwards and downwards (see e.g. figure 2b).

The length of the flanges may vary. The length of the flanges may depend on the trocar to be utilized and the surgery to be performed.

In exemplary embodiments, the length, l, of each one of the projecting flange portion may be in the range of from 20 to 70 mm, preferably from 25 to 50 mm.

The length, l, is measured from the distal side of the dressing to a distal endpoint of the flange. Accordingly, the length of the projecting flange portion relates to the "free" and non-attached part of the flange.

The distal end portion may have a length corresponding to from 10 to 50 % of the total length of the projecting flange portion.

The middle portion may have a length corresponding to 50 to 90% of the total length of the projecting flange portion.

In exemplary embodiments, and as illustrated in figure 1b, the aperture 103 may be covered by an incision film 109 configured to be cut prior to or during insertion of a trocar into the aperture 103.

As used herein, the term "incision film" means a film used during surgery, and which is designed to be cut through. The incision film, which may also be referred to as a "surgical incision film" improves the sterility at the surgical site and prevents leakage of carbon dioxide from the body cavity.

The incision film may be attached to a portion of the adhesive skin contact layer. Alternatively, the incision film may be arranged between the backing layer and the adhesive skin contact layer. The incision film comprises a proximal side facing the skin of a patient, and an opposing distal side facing away from the skin of a patient. The proximal side of the incision film may be adhesive or non-adhesive.

The incision film may e.g. comprise polyurethane, polyester and/or polypropylene

As illustrated in figure 1b, the incision film 109 may comprise markings indicating where and/or how to cut the incision film 109.

One challenge during MIS procedures is to puncture the skin of a patient correctly prior to insertion of the trocar. If a too large incision is made in the skin, carbon dioxide inflated into the body cavity prior to surgery may escape from the incision. If a too small incision is made, it may be difficult and harmful to the patient when the trocar is to be inserted.

The markings indicate to the caregiver the correct length of the incision; i.e. a length that matches the diameter of a trocar cannula. In figure 1b, the markings 110 are provided in the form of a dotted circle. The size of the circle matches the size of a conventional trocar cannula. The surgeon may cut through the incision film along the diameter of the circle.

The markings are not limited to the dotted circular illustrated in figure 1b, but any markings that guides the caregiver to correctly puncture the skin may be utilized.

The adhesive skin contact layer may comprise any skin-compatible adhesive.

Preferably, the adhesive skin contact layer 102 comprises a silicone-based adhesive.

A silicone-based adhesive is gentle to the skin and may be removed and applied to the skin in a gentle manner, without causing any trauma. For example, the adhesive skin contact layer 102 may comprise a silicone gel. The silicone gel may be provided as a coating on the backing layer 101 or a pad, if present.

The adhesive skin contact layer 102 may comprise one or more sub-layers. For example, the adhesive skin contact layer 102 may comprise a polymeric film and an adhesive silicone gel layer, wherein the adhesive silicone gel layer is arranged to contact the skin.

The adhesive skin contact layer 102 preferably comprises a plurality of perforations. The perforations facilitate the entry of wound exudate into the dressing.

As illustrated in the zoomed-in view in figure 1b, the dressing may comprise an absorbent pad 111 between the backing layer 101 and the adhesive skin contact layer 102.

The absorbent pad 111 keeps the area circumventing the incision and the inserted trocar free from blood. The absorbent pad 111 absorbs the blood and prevents maceration at the surgical site. Furthermore, the absorbent pad prevents contaminating microorganisms from accumulating at the skin or incision site. In embodiments where the dressing comprises a pad, the backing layer 101 may be adhesively attached to the pad. Alternatively, the backing layer 101 may be laminated to the pad. For example, heat lamination may be utilized to apply the backing layer 101 to the absorbent pad 111.

The absorbent pad 111 preferably comprises a polyurethane foam. Typically, the polyurethane foam is a hydrophilic polyurethane foam. A polyurethane foam has a pressure relieving effect and is capable of absorbing large amounts of fluids. The absorbent pad 111 may comprise one or more layers. If the pad comprises a plurality of pad-forming layers, the pad-forming layers may be laminated or attached to each other.

In embodiments where the medical dressing comprises an absorbent pad, the absorbent pad comprises a centrally disposed aperture, wherein the aperture of the absorbent pad is aligned with the aperture of the backing layer and the adhesive skin contact layer. The aperture of the dressing may extend through the backing layer, absorbent pad and the adhesive skin contact layer (as illustrated in the figures).

The surface area of the backing layer 101 typically corresponds to the surface area of the adhesive skin contact layer 102. Accordingly, the backing layer and the adhesive skin contact layer are co-extensive.

The absorbent pad may have a surface area corresponding to the surface area of the backing layer and the adhesive skin contact layer.

Alternatively, as illustrated in the figures, the absorbent pad may have a smaller surface area than the backing layer 101 and the adhesive skin contact layer 102. Accordingly, the backing layer and the adhesive skin contact layer 102 may extend beyond the contour of the absorbent pad to form a border portion. This construction may be beneficial to improve the adhesion to the skin and prevent the dressing from detaching from the skin.

The medical dressing of the present disclosure is to be used in conjunction with a trocar.

A trocar system 200 comprising a medical dressing 100 as defined hereinabove and a trocar 201 is conceptually illustrated in figures 2a and 2b.

As used herein, the term "trocar" means a device through which a minimal invasive surgery can be accomplished. The trocar typically comprises at least a cannula (see 202 in figure 2a); i.e. a hollow tube shaped member, and a gas-tight valve (see 203 in figure 2a). The trocar allows for the entry of surgical instruments, such as graspers, scissors, staplers, cameras etc.

The trocar 201 comprises a cannula 202. The cannula serves as the "working channel" through which the surgical tools are inserted.

The cannula 202 is typically a hollow tube made of plastic or metal. The cannula may comprise a gas-tight valve 203 that provides for an internal air-seal, allowing instruments to move in and out of the cannula without loss of pneumoperitoneum; i.e. gas present in the body cavity. The valve may be manually or automatically retractable during instrument passage.

The tip of the cannula may be conical, blunt, eccentric or pyramidal to facilitate penetration and entry into a body cavity.

The exterior surface of the cannula may be threaded or flat.

As illustrated in figure 2a, the medical dressing 100 is attached to the skin 114 of a patient in an area where the MIS procedure is to be accomplished. After attachment of the medical dressing 100, the surgeon may puncture the skin 114 through the aperture 103 of the medical dressing. This may be performed by means of a scalpel or any puncturing tool.

As explained hereinbefore, in cases where the aperture 103 is covered by an incision film, the surgeon may puncture the skin through the incision film covering the aperture. Thereafter, a trocar 201 may be inserted into the incise. Accordingly, the dressing 100 surrounds the trocar 201; i.e. the trocar cannula 202 during use.

When the trocar 201 has been inserted into the body cavity, the release liner portions 108a-d of the flanges 104a-d are removed, as illustrated by the arrows in figure 2a. The adhesive distal end portions of the flanges may subsequently be attached to the trocar cannula 202.

Figure 2b illustrates the scenario where the distal end portions are attached to the cannula 202. As indicated by the arrow, the flanges 104a-d facilitate the movement of the trocar 201 downwards, and upwards, respectively. The flanges 104a-d may adopt a folded or bent configuration when the trocar 201 is moved downwards, and an extended configuration when the trocar 201 is moved upwards. The flanges 104a-d also allow the surgeon to tilt the trocar 201 such that the surgeon may adjust the angular position of the trocar depending on his/her position in the room and depending on the specific surgical situation.

In some cases, it may be desired to insert and maintain the trocar in a tilted configuration. The flanges may then be attached to the trocar cannula at different heights on the cannula.

After the surgery is completed, the trocar system 200 may be removed from the body cavity and the skin of the patient in a simple and gentle manner. The distal end portions of the flanges 104a-d may first be detached from the trocar cannula 202, and the trocar 201 may then be exerted from the body cavity. Subsequently, the dressing 100 may be removed from the skin.

Alternatively, the dressing 100 and the trocar 201 may be removed simultaneously.

Compared to conventional means to detach and remove a fixed trocar from a surgical site, the dressing (and system) of the present disclosure provides for a safer and more simple solution for the patient and the surgical staff. No additional fixation or stabilization means is required inside the body cavity (as is the case with balloon trocars) or secured to the skin (as is the case with Hasson trocars).

As illustrated in figures 1a and 1b, the dressing may further comprise a gripping tab 112 to facilitate the removal of the dressing from the skin. The gripping tab 112 may be formed from the backing layer 101 and/or the adhesive skin contact layer 102. The gripping tab may form a protruding portion extending beyond the perimeter of the backing layer. As illustrated in figures 1a and 1b, the gripping tab 112 may project radially from an outer edge of the backing layer (and the dressing).

Accordingly, the attachment and detachment of the dressing and the trocar can be carried out in a fast and simple manner.

The gripping tab 112 may comprise a proximal side facing the skin of a patient and an opposing distal side facing away from the skin of a patient. The proximal side of the gripping tab 112 may be adhesive or non-adhesive.

Although the medical dressing 100 is illustrated as being circular in the figures, it must be understood that the dressing of the present disclosure is not limited to a particular shape. Any dressing shape is conceivable, e.g. square, oval, rectangular or circular any other shape

The medical dressing may be formed integrally. As illustrated in figures 1a, 2a and 2b, the dressing 100 extends uninterrupted around the aperture 103. The dressing may thus form a closed ring around the aperture 103 and the trocar cannula during use.

This may be beneficial to secure a tight seal to the skin and also to prevent contaminants from entering the incision or the area of the skin circumventing the incision

Alternatively, as illustrated in figure 1b, the dressing may comprise a cutting line 113 extending through the layers of the dressing from a peripheral edge of the dressing to an edge of the aperture 103.

The cutting line 113 may further facilitate the removal of the dressing 100 from the skin. It also allows the dressing to be removed and replaced during surgery; i.e. when the trocar is still inserted into the body cavity. This may be beneficial in scenarios where a lot of blood is exuded; i.e. when the dressing is fully saturated.

The medical dressing 100 may further comprise a release liner (not shown) detachably attached to the adhesive skin contact layer 102. The release liner prevents contamination of the adhesive skin contact layer 102 and is removed prior to application of the dressing to the skin. The release liner portion may comprise the same material as the release liner portions attached to the distal end portions of the flanges, as described hereinbefore.

In exemplary embodiments, the medical dressing comprises an antimicrobial compound.

Accordingly, contaminating microorganisms present at the surgical site or at the skin surrounding the surgical site may be efficiently eradicated. Growth of infecting microorganisms is thereby prevented, which avoids colonization at the surgical site and within the dressing.

For example, the adhesive skin contact layer may comprise a coating comprising an antimicrobial compound.

Alternatively, or in addition, the absorbent pad, where present, may comprise an antimicrobial compound.

It is also conceivable that the incision film comprises a coating comprising an antimicrobial compound.

For example, the antimicrobial compound may be selected from the group comprising a silver salt, a chlorhexidine salt, polyhexamethylene biguaninde, benzethonium chloride, polidiallyldimethylammonium chloride etc.

The trocar system 200 of the present disclosure may be provided as a kit comprising a trocar 201 and a medical dressing 100 as described hereinabove. The kit may comprise additional components, such as surgical instruments to be used for a specific surgery. The kit may also comprise a scalpel or a puncturing tool to puncture the skin prior to insertion of the trocar.

In cases where the medical dressing comprises an incision film with markings, the markings may be matched to the diameter of the trocar comprised in the kit.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (100) for use in conjunction with a trocar (201), wherein said dressing (100) has a distal side provided with a backing layer (101) and an opposing proximal side provided with an adhesive skin contact layer (102); said dressing (100) comprising a centrally disposed aperture (103) for receiving a trocar (201) during use, wherein said aperture (103) extends through said backing layer (101) and said adhesive skin contact layer (102); said dressing (100) further comprising at least a first flange (104a) and a second flange (104b) configured to project upwardly from said distal side of said dressing; wherein each one of said at least first (104a) and second (104b) flanges comprises a respective proximal end portion (105a-c) being attached to said dressing (100) and a respective distal end portion (106a-d) configured to be adhesively attached to a trocar (201) during use.

2. The medical dressing (100) according to claim 1, wherein said dressing (100) further comprises at least a third (104c), e.g. at least a fourth (104d) flange projecting upwardly from said distal side of said dressing (100).

3. The medical dressing (100) according to claim 1 or claim 2, wherein each one of said flanges comprises a foldable material.

4. The medical dressing (100) according to any one of the preceding claims, wherein said flanges (104a-d) are disposed symmetrically along the perimeter of said aperture (103).

5. The medical dressing (100) according to any one of the preceding claims, wherein each one of said flanges (104a-d) comprises a middle portion (107a-d) arranged between said proximal end portion (105a-c) and said distal end portion (106a-d); said middle portion and said distal end portion forming a respective projecting flange portion being unattached to said dressing (100).

6. The medical dressing (100) according to any one of the preceding claims, wherein said distal end portion (106a-d) of each one of said flanges (104a-d) comprises an adhesive coating.

7. The medical dressing (100) according to claim 6, wherein each one of said flanges (104a-d) further comprises a release liner portion (108a-d) attached to said adhesive coating of said distal end portion (106a-d).

8. The medical dressing (100) according to claim 5 or any one of claims 6-7 when dependent on claim 5, wherein said middle portion (107a-d) of each one of said flanges (104a-d) is non-adhesive.

9. The medical dressing (100) according to claim 5 or any one of claims 6-8 when dependent on claim 5, wherein the length, l, of each one of said projecting flange portion is in the range of from 20 to 70 mm, preferably from 25 to 50 mm.

10. The medical dressing (100) according to any one of the preceding claims, wherein the proximal end portion (105a-c) of each one of said flanges (104a-d) is attached to said backing layer (101) of said dressing (100) or is attached to said dressing between said backing layer (101) and said adhesive skin contact layer (102).

11. The medical dressing (100) according to any one of the preceding claims, wherein said aperture (103) is covered by an incision film (109) configured to be cut prior to or during insertion of a trocar (201) into said aperture (103).

12. The medical dressing (100) according to claim 11, wherein said incision film (109) comprises markings (110) indicating where and/or how to cut said incision film (109).

13. The medical dressing (100) according to any one of the preceding claims, wherein said adhesive skin contact layer (102) comprises a silicone-based adhesive.

14. The medical dressing (100) according to any one of the preceding claims, wherein said medical dressing comprises an absorbent pad (111) between said backing layer (101) and said adhesive skin contact layer (102).

15. A trocar system (200) comprising the medical dressing (100) according to any one of the preceding claims and a trocar (201).

## Patentansprüche

1. Medizinischer Verband (100) zur Verwendung in Verbindung mit einem Trokar (201), wobei der Verband (100) eine distale Seite, die mit einer Trägerschicht (101) versehen ist, und eine gegenüberliegende proximale Seite aufweist, die mit einer haftenden Hautkontaktschicht (102) versehen ist; wobei der Verband (100) eine mittig angeordnete Öffnung (103) zum Aufnehmen eines Trokars (201) während des Gebrauchs umfasst, wobei die Öffnung (103) durch die Trägerschicht (101) und die haftende Hautkontaktschicht (102) verläuft; wobei der Verband (100) ferner mindestens einen ersten Bund (104a) und einen zweiten Bund (104b) umfasst, die so eingerichtet sind, dass sie von der distalen Seite des Verbands aus nach oben ragen; wobei sowohl der mindestens eine erste (104a) als auch zweite (104b) Bund einen jeweiligen proximalen Endabschnitt (105a-c), der an dem Verband (100) befestigt ist, und einen jeweiligen distalen Endabschnitt (106a-d) umfasst, der so eingerichtet ist, dass er während des Gebrauchs haftend an einem Trokar (201) angebracht ist.

2. Medizinischer Verband (100) nach Anspruch 1, wobei der Verband (100) ferner mindestens einen dritten (104c), z.B. mindestens einen vierten (104d) Bund umfasst, der von der distalen Seite des Verbands (100) aus nach oben ragt.

3. Medizinischer Verband (100) nach Anspruch 1 oder Anspruch 2, wobei jeder der Bünde ein faltbares Material umfasst.

4. Medizinischer Verband (100) nach einem der vorhergehenden Ansprüche, wobei die Bünde (104a-d) symmetrisch entlang dem Umfang der Öffnung (103) angeordnet sind.

5. Medizinischer Verband (100) nach einem der vorhergehenden Ansprüche, wobei jeder der Bünde (104a-d) einen mittleren Abschnitt (107a-d) umfasst, der zwischen dem proximalen Endabschnitt (105a-c) und dem distalen Endabschnitt (106a-d) angeordnet ist; wobei der mittlere Abschnitt und der distale Endabschnitt einen jeweiligen vorstehenden Bundabschnitt bilden, der nicht an dem Verband (100) angebracht ist.

6. Medizinischer Verband (100) nach einem der vorhergehenden Ansprüche, wobei der distale Endabschnitt (106a-d) von jedem der Bünde (104a-d) eine Klebstoffbeschichtung umfasst.

7. Medizinischer Verband (100) nach Anspruch 6, wobei jeder der Bünde (104a-d) ferner einen Trennpapierabschnitt (108a-d) umfasst, der an der Klebstoffbeschichtung des distalen Endabschnitts (106a-d) angebracht ist.

8. Medizinischer Verband (100) nach Anspruch 5 oder einem der Ansprüche 6-7 bei Abhängigkeit von Anspruch 5, wobei der mittlere Abschnitt (107a-d) von jedem der Bünde (104a-d) nicht haftend ist.

9. Medizinischer Verband (100) nach Anspruch 5 oder einem der Ansprüche 6-8 bei Abhängigkeit von Anspruch 5, wobei die Länge, 1, von jedem vorstehenden Bundabschnitt im Bereich von 20 bis 70 mm, vorzugsweise von 25 bis 50 mm liegt.

10. Medizinischer Verband (100) nach einem der vorhergehenden Ansprüche, wobei der proximale Endabschnitt (105a-c) von jedem der Bünde (104a-d) an der Trägerschicht (101) des Verbands (100) angebracht ist oder zwischen der Trägerschicht (101) und der haftenden Hautkontaktschicht (102) an dem Verband angebracht ist.

11. Medizinischer Verband (100) nach einem der vorhergehenden Ansprüche, wobei die Öffnung (103) mit einer Inzisionsfolie (109) abgedeckt ist, die so eingerichtet ist, dass sie vor dem Einführen oder während des Einführens eines Trokars (201) in die Öffnung (103) eingeschnitten wird.

12. Medizinischer Verband (100) nach Anspruch 11, wobei die Inzisionsfolie (109) Kennzeichnungen (110) umfasst, mit denen angegeben ist, wo und/oder wie die Inzisionsfolie (109) einzuschneiden ist.

13. Medizinischer Verband (100) nach einem der vorhergehenden Ansprüche, wobei die haftende Hautkontaktschicht (102) einen Klebstoff auf Silikonbasis umfasst.

14. Medizinischer Verband (100) nach einem der vorhergehenden Ansprüche, wobei der medizinische Verband ein absorbierendes Kissen (111) zwischen der Trägerschicht (101) und der haftenden Körperkontaktschicht (102) umfasst.

15. Trokarsystem (200), das den medizinischen Verband (100) nach einem der vorhergehenden Ansprüche und ein Trokar (201) umfasst.

## Revendications

1. Pansement médical (100) destiné à être utilisé conjointement avec un trocart (201), ledit pansement (100) ayant un côté distal pourvu d'une couche de support (101) et un côté proximal opposé pourvu d'une couche adhésive de contact avec la peau (102), ledit pansement (100) comprenant une ouverture centrale (103) destinée à recevoir un trocart (201) pendant l'utilisation, ladite ouverture (103) s'étendant à travers ladite couche de support (101) et ladite couche adhésive de contact avec la peau (102) ; ledit pansement (100) comprenant en outre au moins une première bride (104a) et une deuxième bride (104b) configurées pour faire saillie vers le haut à partir dudit côté distal dudit pansement ; chacune desdites première (104a) et seconde (104b) brides comprenant une section d'extrémité proximale correspondante (105a-c) fixée audit pansement (100) et une section d'extrémité distale respective (106a-d) configurée pour être fixée de manière adhésive à un trocart (201) pendant l'utilisation.

2. Pansement médical (100) selon la revendication 1, dans lequel ledit pansement (100) comprend en outre au moins une troisième (104c), par exemple au moins une quatrième (104d) bride, faisant saillie vers le haut à partir dudit côté distal dudit pansement (100).

3. Pansement médical (100) selon la revendication 1 ou la revendication 2, dans lequel chacune desdites brides comprend un matériau pliable.

4. Pansement médical (100) selon l'une quelconque des revendications précédentes, dans lequel lesdites brides (104a-d) sont disposées symétriquement le long du périmètre de ladite ouverture (103).

5. Pansement médical (100) selon l'une quelconque des revendications précédentes, dans lequel chacune desdites brides (104a-d) comprend une section médiane (107a-d) disposée entre ladite section d'extrémité proximale (105a-c) et ladite section d'extrémité distale (106a-d) ; ladite section médiane et ladite section d'extrémité distale formant une section de collerette saillante respective n'étant pas fixées a audit pansement (100).

6. Pansement médical (100) selon l'une quelconque des revendications précédentes, dans lequel ladite section d'extrémité distale (106a-d) de chacune desdites brides (104a-d) comprend un revêtement adhésif.

7. Pansement médical (100) selon la revendication 6, dans lequel chacune desdites brides (104a-d) comprend en outre une section de doublure détachable (108a-d) fixée audit revêtement adhésif de ladite section d'extrémité distale (106a-d).

8. Pansement médical (100) selon la revendication 5 ou l'une quelconque des revendications 6-7 lorsqu'elles dépendent de la revendication 5, dans lequel ladite section médiane (107a-d) de chacune desdites brides (104a-d) est non adhésive.

9. Pansement médical (100) selon la revendication 5 ou l'une quelconque des revendications 6-8 lorsqu'elles dépendent de la revendication 5, dans lequel la longueur, 1, de chacune desdites sections de brides saillantes est dans la plage de 20 à 70 mm, de préférence de 25 à 50 mm.

10. Pansement médical (100) selon l'une quelconque des revendications précédentes, dans lequel la section d'extrémité proximale (105a-c) de chacune desdites brides (104a-d) est fixée à ladite couche de support (101) dudit pansement (100) ou est fixée audit pansement entre ladite couche de support (101) et ladite couche adhésive de contact avec la peau (102).

11. Pansement médical (100) selon l'une quelconque des revendications précédentes, dans lequel ladite ouverture (103) est recouverte par un film à inciser (109) configuré pour être coupé avant ou pendant l'insertion d'un trocart (201) dans ladite ouverture (103) .

12. Pansement médical (100) selon la revendication 11, dans lequel ledit film à inciser (109) comprend des marquages (110) indiquant où et/ou comment couper ledit film à inciser (109).

13. Pansement médical (100) selon l'une quelconque des revendications précédentes, dans lequel ladite couche adhésive de contact avec la peau (102) comprend un adhésif à base de silicone.

14. Pansement médical (100) selon l'une quelconque des revendications précédentes, dans lequel ledit pansement médical comprend un tampon absorbant (111) entre ladite couche de support (101) et ladite couche adhésive de contact avec la peau (102).

15. Système de trocart (200) comprenant le pansement médical (100) selon l'une quelconque des revendications précédentes et un trocart (201).
